# EUROPEAN PATENT SPECIFICATION

(11) **EP 0 395 144 B1**
(45) Date of publication and mention of the grant of the patent: **30.06.1993**
(21) Application number: 90200959.6
(22) Date of filing: 18.04.1990
(51) Int. Cl.: C07C 273/18, C07C 275/28

(54) **A process for the preparation of aromatic ureas**
Verfahren zur Herstellung von aromatischen Harnstoffen
Procédé de préparation d'urées aromatiques

(30) Priority: 19.04.1989 GB 8908869
(43) Date of publication of application: 31.10.1990
(73) Proprietor: SHELL INTERNATIONALE RESEARCH MAATSCHAPPIJ B.V., 2596 HR Den Haag (NL)
(72) Inventor: Goodall, Brian Leslie, Akron, Ohio 44313 (US); Terlouw, Willem, NL-1031 CM Amsterdam (NL)

(56) References cited:
- EP-A- 0 086 281
- EP-A- 0 231 045
- EP-A- 0 235 865
- EP-A- 0 250 037
- EP-A- 0 319 111
- JOURNAL OF ORGANOMETTALIC CHEMISTRY, vol. 336, 1987 ; P. GIONNOCCARO : "Palladium-catalysed N,N-disubstituted urea synthesis by oxidative carbonylation of amines under CO and O2 at atmospheric pressure"

## Description

The present invention relates to a process for the preparation of aromatic ureas and to aromatic ureas prepared by means of such a process.

Aromatic ureas are a known class of commercially available compounds, finding particular use as herbicides. Hitherto, processes for the manufacture of aromatic ureas have frequently required the use of environmentally unacceptable compounds, for example phosgene. There is, therefore, a need to provide a process for preparing aromatic ureas that is not reliant on the use of such compounds.

P. Giannoccaro (Journal of Organometallic Chemistry, 336 (1987), 271-278) has proposed a process for the preparation of N,N'-disubstituted ureas in which aromatic and aliphatic primary amines in alcohol solution are reacted with carbon monoxide and oxygen under mild conditions in the presence of catalytic amounts of palladium (II) chloride or a palladium (II) complex. Under more severe conditions of temperature and pressure carbamate esters are said to be obtained. The best yields are stated to have been obtained using a catalyst system comprising copper (II) chloride as a co-catalyst.

In relation to the preparation of carbamate esters, Japanese patent application publication No. 60-139 659 (JP-A-60-139 659) discloses a process for preparing urethanes (carbamates) in which a primary or secondary amine is caused to react with carbon monoxide and an organic hydroxyl compound in the presence of an oxidising agent and a catalyst comprising a platinum-group metal or platinum-group metal compound, at least one halogen compound and at least one chelating reagent. A number of drawbacks of using a catalyst comprising a halogen compound are discussed in the specification of JP-A-60-139 659. For example, chlorides are said to be responsible for a large amount of corrosion in reaction vessels and pipework and are difficult to remove from the products of the reaction. However, the specification discloses that the halogen compounds play an important role in the reaction by acting as promoters. Therefore, in spite of the drawbacks associated with the use of halogen compounds, the processes disclosed and exemplified in JP-A-60-139 659 all rely on the use of a catalyst comprising a halogen compound as a promoter or co-catalyst. Platinum-group metals listed in the specification of JP-A-60-139 659 as being for use in the catalyst are palladium, rhodium, platinum, ruthenium, iridium and osmium, with platinum and rhodium being especially preferred. Halogen compounds may be either organic or inorganic, with metal halides being preferred. Possible metal halides for use in the process include halides of alkali metals, alkaline-earth metals, copper, silver, zinc, cadmium, mercury, aluminium, potassium, thallium, germanium, tin, lead, antimony, bismuth, titanium, zirconium, vanadium, niobium, tantalum, tellurium, chromium, molybdenum, tungsten, manganese, rhenium, iron, cobalt, nickel and rare-earth metals. Halides of alkali metals and alkaline-earth metals are particularly preferred. Chelating reagents for use in the process of JP-A-60-139 659 are described as being compounds having two or more coordinate groups in one ligand. One of the coordinate groups is preferably a tertiary amino group, a nitrogen-containing aromatic ring or an oxime. Oxidising agents are preferably molecular oxygen and/or organic nitro compounds, with molecular oxygen being especially preferred.

Further reliance on the use of metal halides as co-catalysts or promotors can be found, for example, in Japanese patent application publication No. 58-144 363 (JP-A-58-144 363) which discloses a process for the preparation of ureas comprising reacting a primary or secondary amine with carbon monoxide in the presence of oxygen using a catalyst system containing at least one of the platinum group of metals or compounds thereof and at least one halogen compound. Preferred platinum group metals are said to be palladium and rhenium. Preferred halogen compounds are stated to be metal halides, halogenated onium compounds or compounds capable of producing halogenated onium compounds, or organic halogenises. A catalyst system comprising palladium black and cesium iodide is specifically exemplified.

Surprisingly, it has been found that a palladium catalyst which comprises a ligand and which, contrary to the teaching of the prior art discussed above, is not reliant on the presence of a halide as a co-catalyst can be used to prepare certain ureas in high yields, whilst at the same time overcoming the problems associated with using a metal halide as co-catalyst.

According to the present invention there is provided a process for preparing aromatic ureas having the general formula I: 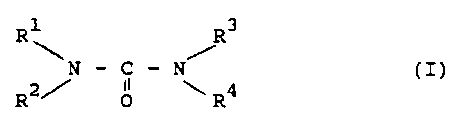
wherein each of R¹ and R³ independently represents a hydrogen atom, or an alkyl or cycloalkyl group, R² represents an optionally substituted aryl or heteroaryl group, and R⁴ represents an alkyl, alkenyl, alkynyl or alkoxy group, or an optionally substituted cycloalkyl, aryl or heteroaryl group, which process comprises reacting an aromatic amine having the general formula II: 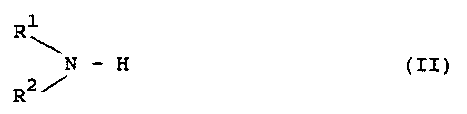
wherein R¹ and R² are as hereinbefore defined, or a symmetrical aromatic urea having the general formula III: 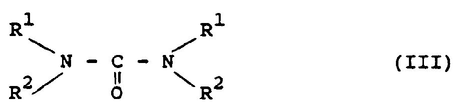
wherein R¹ and R² are as hereinbefore defined, with an amine having the general formula IV: 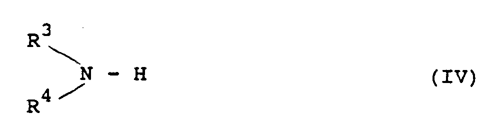
wherein R³ and R⁴ are as hereinbefore defined, carbon monoxide and oxygen in the presence of a catalyst comprising:
a) palladium;
b) an organo-nitrogen or organo-phosphorous ligand having a lone pair of electrons; and
c) a metal salt comprising a cation of a metal selected from the group consisting of copper, iron, vanadium, chromium, zinc, tin, uranium and cerium, and an anion of an acid having a pK of less than 2, except of a hydrohalogenic acid.

The starting materials for the process of the present invention may comprise an aromatic amine or a symmetrical aromatic urea or a combination of an amine and its corresponding symmetrical urea.

Alkyl and alkoxy groups preferably have from 1 to 12 carbon atoms, especially 1 to 6 carbon atoms. Alkenyl and alkynyl groups preferably have from 2 to 12 carbon atoms, especially 2 to 6 carbon atoms. Cycloalkyl groups preferably have from 3 to 10 carbon atoms, especially 3 to 8 carbon atoms. Alkyl, alkoxy, alkenyl and alkynyl groups may be straight chain or branched.

Optional substituents for cycloalkyl, aryl and heteroaryl groups may be independently selected from halogen atoms, especially fluorine, chlorine and bromine and alkyl, haloalkyl, alkoxy, carboxy, carbonyloxy, aminocarbonyloxy groups, and aryl and heteroaryl groups themselves optionally substituted by one or more substituents selected from halogen atoms and alkyl, haloalkyl and alkoxy groups. Alkyl, haloalkyl and alkoxy groups when present as substituents preferably have from 1 to 4 carbon atoms especially 1 or 2 carbon atoms.

Preferably each of R¹ and R³ independently represents a hydrogen atom, a C₁₋₄ alkyl group or a C₃₋₅ cycloalkyl group. Most preferably each of R¹ and R³ independently represents a hydrogen atom or methyl.

Preferably R² represents an optionally substituted phenyl group or nitrogen-containing heteroaryl group, for example isoxazolyl, triazinyl and oxadiazolyl. Most preferably R² represents a phenyl group bearing one or more substituents selected from halogen atoms, especially chlorine and bromine, alkyl, especially methyl, alkoxy, especially methoxy, and haloalkyl, especially trifluoromethyl.

R⁴ preferably represents an alkyl, alkoxy, alkenyl or alkynyl group or an optionally substituted cycloalkyl or phenyl group. Most preferably R⁴ represents a methyl or methoxy group or a phenyl group bearing one or more substituents selected from halogen atoms, especially chlorine or bromine, alkyl, especially methyl, alkoxy, especially methoxy and haloalkyl, especially trifluoromethyl.

Aromatic ureas of particular commercial significance are those having herbicidal activity. Examples of such aromatic ureas include N'-(4-chlorophenyl)-N,N-dimethylurea, N'-(3-chloro-4-methylphenyl)-N,N-dimethylurea, N'-[4-(4-methoxyphenoxy)phenyl]-N,N-dimethylurea, N'-(3,4-dichlorophenyl)-N,N-dimethylurea, N'-[5-(1,1-dimethylethyl)-3-isoxazolyl]-N,N-dimethylurea, N'-(3,4-dichlorophenyl)-N-methoxy-N-methylurea, N'-(4-bromophenyl)-N-methoxy-N-methylurea, N'-(3,4-dichlorophenyl-N-butyl-N-methylurea, N-[(4-chlorophenyl)methyl]-N-cyclopentyl-N'-phenylurea, N-(2-methylcyclohexyl)-N'-phenylurea.
Therefore, the process of the present invention is particularly useful when the aromatic amine, and/or symmetrical aromatic urea, and the amine are selected to produce such a compound.

The aromatic amines, symmetrical ureas, and primary and secondary amines used as starting materials in the process according to the present invention are either commercially available compounds or can be readily obtained by standard preparative methods.

The process of the present invention is carried out in the presence of a catalyst comprising palladium. The palladium may be present in the form of palladium metal, preferably deposited on an inert carrier, for example alumina or carbon.
Alternatively, the palladium may be present as palladium compounds, in particular complexes or salts of palladium. If the palladium is present as a palladium compound, the compound is preferably soluble in the reaction mixture.

Suitable palladium salts include palladium chloride, palladium bromide, palladium iodide and palladium carboxylates, for example palladium acetate, palladium propionate and palladium isobutyrate. The preferred salts are palladium carboxylates. Especially preferred is palladium acetate. Examples of suitable complexes of palladium include palladium acetylacetonate, sodium tetrachloropalladate, potassium tetrachloropalladate, and potassium tetraiodopalladate.

The quantity of palladium used in the process is not critical, but is conveniently in the range of 0.001% w/w to 10% w/w palladium metal and/or palladium compound, in particular in the range of 0.005% w/w to 3% w/w palladium metal and/or palladium compound, calculated on the amount of aromatic amine or symmetrical aromatic urea present.

The catalytic system further comprises an organo-nitrogen or organo-phosphorous ligand having a lone pair of electrons.

Suitable organo-phosphorous ligands which may be used in the process of the present invention include phosphites and tertiary phosphines of the general formula P.OR^{a}.OR^{b}.OR^{c} or PR^{a}R^{b}R^{c}, wherein each of R^{a}, R^{b} and R^{c} independently represents an alkyl, cycloalkyl, aryl, alkaryl or aralkyl group each having up to 10 carbon atoms, optionally substituted by one or more substituents selected from fluorine and chlorine atoms, alkoxy and aryloxy groups each having up to 10 carbon atoms, in particular methoxy and phenoxy groups, cyano groups, and groups of formula -PR^{a}R^{b}; or R^{a} and R^{b} together with the interjacent heteroatom together represent a heteroaryl group. Preferred monodentate organophosphorus compounds include trimethylphosphine, triethylphosphine, tri-n-butylphosphine and triphenylphosphine. Examples of suitable bidentate phosphorus-containing ligands are 2-dimethyl-2-(methyldiphenylphosphino)-1,3-di(diphenylphosphino)propane, tetramethyl diphosphinoethane, tetramethyl diphosphinopropane, tetraethyl diphosphinoethane, tetrabutyl diphosphinoethane, dimethyl diethyl diphosphinoethane, tetraphenyl diphosphinoethane, tetraperfluorophenyl diphosphinoethane, tetraphenyl diphosphinopropane, tetraphenyl diphosphinobutane, dimethyl diphenyl diphosphinopropane, tetratolyl diphosphinoethane, ditolyl diphenyl diphosphinoethane, tetratrifluoromethyl diphosphinotetrafluoroethane, tetraphenyl diphosphinoethene and derivatives thereof and 1,2-bis-(diphenylphosphino)benzene and derivatives thereof. Preferred are tetraphenyldiphosphinobutane, tetraphenyl diphosphinoethane and tetraphenyl diphosphinopropane.

Organo-nitrogen ligands which may conveniently be used in the process of the present invention include mono-, bi-, or poly-cyclic systems containing one or more nitrogen atoms. Systems containing 1 or 2 nitrogen atoms are preferred. Such cyclic organo-nitrogen compounds are preferably those having the general formula V or VI: 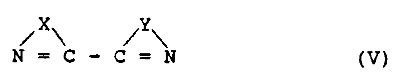 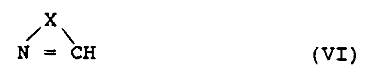
wherein each of X and Y, which, in formula V, may be the same or different, represents a bridging group containing 3 or 4 atoms in the bridge of which at least 2 are carbon atoms. Any atoms in the bridging groups X and Y other than carbon atoms are preferably nitrogen atoms. In compounds having the general formula V, X and Y may be bound to each other by means of a connection in addition to that already formed by the carbon atoms shown in formula V. X and Y in formula V are preferably the same. The cyclic systems of formula V and VI are preferably aromatic. Preferred organo-nitrogen ligands are those having the general formula V.

Specific examples of suitable organo-nitrogen compounds include pyrrole, pyridine, pyrimidine, phenanthroline, pyrazine, benztriazole, 2,2'-dipyridyl, 2,2'-biquinoline, bis-pyridylketone, bis-pyridylglyoxal, and their alkyl-substituted derivatives. Analogous compounds containing other substituents, for example sulphonyl, carbonyl, alkoxy and halide moieties may also be used. The preferred compounds are 1,10-phenanthroline, 2,2'-dipyridyl, pyridine and alkyl-substituted pyridines such as the various picolines, for example alpha-picoline.

Preferred ligands are bidentate ligands, the nitrogen based ligands being especially preferred. The most preferred ligands are 1,10-phenanthroline and 2,2'-dipyridyl.

The ligand is generally present in a sufficient amount to give a molar ratio of ligand to palladium metal and/or palladium compound of from 0.5:1 to 30:1 preferably of from 5:1 to 20:1.

The catalytic system used in the process of the present invention further comprises a metal salt comprising a cation of a metal selected from the group consisting of copper, iron, vanadium, chromium, zinc, tin, uranium and cerium. The preferred metals are copper, iron, chromium and uranium. Most preferred are the cations of copper, especially the copper (II) cation. The metal salt further comprises an anion of an acid having a pK of less than 2, except an anion of a hydrohalogenic acid. Preferred anions are those of sulphuric acid, fluoroboric acid, p-toluene sulphonic acid and alkyl substituted derivatives thereof, and perchloric acid. The most preferred anion is that of p-toluene sulphonic acid. Other anions that may also be used include those of benzene sulphonic acid, naphthalene sulphonic acid, 2,4,5-trichlorobenzene sulphonic acid, or corresponding bromo- and fluoro-analogues.

Examples of suitable metal salts include copper (I) tosylate, copper (II) tosylate, copper (II) chlorate, iron (II) chlorate, copper (II) fluoroborate, tin (IV) sulphate and uranium (IV) sulphate. The most preferred metal salt is copper (II) tosylate.

Aromatic ureas are prepared by reacting an aromatic amine or a symmetrical aromatic urea with a primary or secondary amine, oxygen and carbon monoxide in the presence of the catalytic system described above.

All of the amine to be reacted may be present, with the aromatic amine or symmetrical aromatic urea, at the start of the reaction. However, it is preferred that the amine is added continuously or portionwise during the period of the reaction. Continuous addition of the amine is especially preferred.

The preferred rate of supply of the amine to the reaction mixture will depend upon the particular reactants and reaction conditions selected, and is readily determined by routine experimentation.

Preferably the amine is supplied over at least 40%, more preferably at least 50% of the period of the reaction. In certain cases it may be advantageous to supply the amine over at least 80%, and even up to 100% of the period of the reaction.

The amine may be supplied in a molar amount less than the molar amount of the aromatic amine, but preferably in a molar amount at least equal to the molar amount of the aromatic amine.

Carbon monoxide and oxygen may be supplied by passing the gases into the reaction mixture during the period of the reaction, preferably throughout the period of the reaction. Oxygen may be supplied in the form of pure oxygen gas or an oxygen-containing stream, most conveniently as air. The oxygen and carbon monoxide streams may be supplied separately to the reaction mixture or, alternatively, may be combined. Typically a combined stream of carbon monoxide and air for supplying to the reaction mixture contains about 85% vol carbon monoxide and about 15% vol air.

The reaction may be performed in the presence of a solvent. Suitable solvents include any unreactive organic solvent, such as halogenated hydrocarbons, for example chloroform, 1,2-dichloroethane, chlorobenzene and the three dichlorobenzenes; hydrocarbons, for example hexane, cyclohexane, octane, benzene, toluene and the three xylenes; ethers, for example diethyl ether, tetrahydrofuran, dioxane and anisole; and esters such as ethyl acetate. Preferred solvents are xylene, chlorobenzene, and the three dichlorobenzenes. A particularly preferred solvent is 1,2-dichlorobenzene.

The process is preferably effected at a temperature up to 175°C. Preferred temperatures are in the range of from 70°C to 160°C, particularly 100°C to 150°C. The process may be carried out at atmospheric pressure except when the desired operating temperature exceeds the boiling temperature of the reaction mixture, when it will be necessary to employ elevated pressures. However, the process is preferably carried out at elevated pressures, for example up to 150 bar, more preferably in the range of from 3 to 50 bar.

The present invention will be further illustrated by the following Examples. Products of the process were identified by high performance liquid chromatography (HPLC) via comparison with authentic samples.

### Preparation of Palladium acetate/copper tosylate/2,2'-dipyridyl catalyst

Copper (II) tosylate was prepared using the following procedure:

P-toluenesulphonic acid (20g) was added to water (500ml) and the resulting solution stirred whilst heating to 50°C. Powdered copper (II) hydroxide was slowly added to the solution during which the pH of the solution was monitored. Addition of the copper (II) hydroxide was stopped when the pH of the solution reached 7 (after the addition of about 10g of hydroxide). The resulting mixture was cooled to ambient temperature (20°C) and filtered. The solvent of the filtrate was evaporated leaving a solid residue. The residue was dried in a vacuum oven at 40°C to give green-blue crystals of copper (II) tosylate containing approximately 10% water in a yield of 21g.

### Example 1

### Preparation of N'-(4-chlorophenyl)-N,N-dimethylurea

Nitrogen was passed through a stainless steel autoclave to render the atmosphere within the autoclave inert. Palladium acetate (0.25mmoles; 0.056g), copper (II) tosylate (0.5mmoles; 0.202g) and 2,2'-dipyridyl (2.5mmoles; 0.42g) were dissolved in 1,2-dichlorobenzene (70ml) and added to the autoclave. 4-chloroaniline (25mmoles; 3.19g) was added and the resulting mixture heated to 130°C. A mixture of 85% vol carbon monoxide and 15% vol air was flushed through the resulting mixture at a pressure of 6 bar and at a flow rate of 6 Nl/hour. The reaction was allowed to proceed for 6 hours, after which time the conversion of 4-chloroaniline was 65%. The supply of the carbon monoxide/air mixture was removed and the resulting mixture was allowed to stand overnight at ambient temperature (20°C). The mixture was reheated to 130°C and a mixture of 85% vol carbon monoxide and 15% vol air once again passed into the reaction mixture at a pressure of 6 bar. A solution of dimethylamine (25mmoles; 1.125g) in 1,2-dichlorobenzene (8ml) was continuously added slowly over a period of 4 hours. After addition of the dimethylamine had been completed, the reaction mixture was stirred for a further 2 hours. After this time, the resulting mixture was analysed by HPLC. The analysis showed a 90% conversion of 4-chloroaniline yielding a product mixture comprising N'-(4-chlorophenyl)-N,N-dimethylurea and N,N'-bis(4-chlorophenyl)urea in a yield of 15mmoles and 2 mmoles respectively.

### Example 2

### Preparation of N'-(5-(1,1-dimethylethyl)-3-isoxazolyl)-N,N-dimethylurea

Nitrogen was passed through a stainless steel autoclave to render the atmosphere within the autoclave inert. Palladium acetate (0.5mmoles; 0.112g), copper (II) tosylate (1mmole; 0.408g) and 2,2'-dipyridyl (5mmoles; 0.84g) were dissolved in 1,2-dichlorobenzene (200ml) and added to the autoclave. 5-(1,1-dimethylethyl)-3-isoxazoleamine (51.8mmoles; 7.25g) was added and the resulting mixture heated to 140°C. A mixture of 85% vol carbon monoxide and 15% vol air was passed into the resulting mixture at a pressure of 11 bar and a flow rate of 6 Nl/hr. A solution of dimethylamine (51mmoles; 2.3g) in 1,2-dichlorobenzene (16ml) was continuously added slowly over a period of 6 hours. Once addition of the dimethylamine solution had been completed, the resulting mixture was analysed by HPLC. The analysis showed a 50% conversion of 5-(1,1-dimethylethyl)-3-isoxazoleamine yielding N'-(5-(1,1-dimethylethyl)-3-isoxazolyl)-N,N-dimethylurea and N,N'-bis (5-(1,1-dimethylethyl)-3-isoxazolyl)urea in a yield of 13 mmoles and 3.5mmoles respectively.

## Claims

1. A process for preparing aromatic ureas having the general formula I: 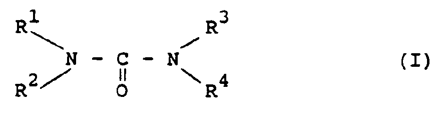 wherein each of R¹ and R³ independently represents a hydrogen atom, an alkyl or cycloalkyl group, R² represents an optionally substituted aryl or heteroaryl group, and R⁴ represents an alkyl, alkenyl, alkynyl or alkoxy group, or an optionally substituted cycloalkyl, aryl or heteroaryl group, which process comprises reacting an aromatic amine having the general formula II: 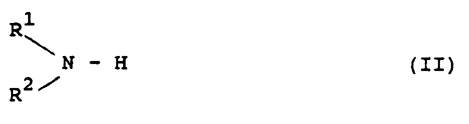 wherein R¹ and R² are as hereinbefore defined, or a symmetrical aromatic urea having the general formula III: 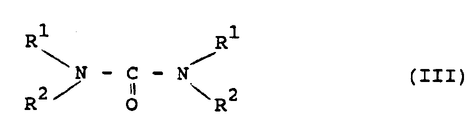 wherein R¹ and R² are as hereinbefore defined, with an amine having the general formula IV: 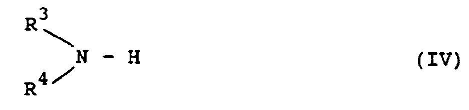 wherein R³ and R⁴ are as hereinbefore defined, carbon monoxide and oxygen in the presence of a catalyst comprising:
a) palladium;
b) an organo-nitrogen or organo-phosphorous ligand having a lone pair of electrons; and
c) a metal salt comprising a cation of a metal selected from the group consisting of copper, iron, vanadium, chromium, zinc, tin, uranium and cerium, and an anion of an acid having a pK of less than 2, except of a hydrohalogenic acid.

2. A process according to claim 1, wherein the catalyst comprises an organo-nitrogen ligand having the general formula V: 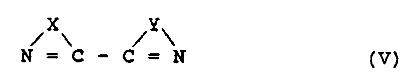 in which X and Y represent the same or different bridging groups each of which has 3 or 4 atoms in the bridge, of which atoms at least two are carbon atoms and which groups X and Y may be bound to each other by means of a connection in addition to that already formed by the carbon atoms shown in formula V.

3. A process according to claim 1 or 2, wherein the catalyst comprises a salt or complex of palladium.

4. A process according to any one of claims 1 to 3, wherein the metal salt comprises a cation of copper.

5. A process according to any preceding claim, wherein the metal salt comprises an anion of the group consisting of sulphate, hydrocarbylsulphonates, fluoroborate and perchlorate.

6. A process according to claim 5, wherein the anion is tosylate.

7. A process according to any preceding claim, wherein the reaction is effected in the presence of a chlorobenzene as solvent.

8. A process according to any preceding claim, wherein the pressure is in the range of from 3 to 150 bar.

9. A process according to any preceding claim, wherein the temperature is in the range of from 70°C to 160°C.

10. A process according to any preceding claim, wherein the carbon monoxide is passed into the reaction mixture continuously during the period of the reaction.

11. A process according to any preceding claim, wherein the amine is supplied continuously or portionwise during the period of the reaction.

12. A process according to claim 11, wherein the amine is supplied over at least 50% of the period of the reaction.

## Patentansprüche

1. Ein Verfahren zur Herstellung aromatischer Harnstoffverbindungen mit der allgemeinen Formel I: 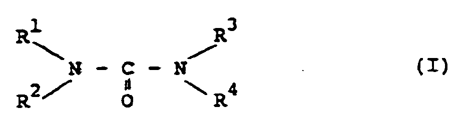 in der jedes R¹ und R³ unabhängig voneinander ein Wasserstoff atom, eine Alkyl- oder Cycloalkylgruppe bedeutet, R² eine gegebenenfalls substituierte Aryl- oder Heteroarylgruppe ist und R⁴ eine Alkyl-, Alkenyl-, Alkynyl- oder Alkoxygruppe oder eine gegebenenfalls substituierte Cycloalkyl-, Aryl- oder Heteroarylgruppe darstellt, welches Verfahren die Reaktion eines aromatischen Amins mit der allgemeinen Formel II: 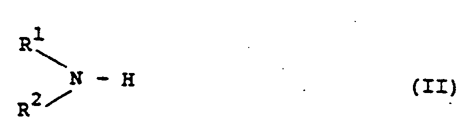 in der R¹ und R² wie vorstehend definiert sind, oder einer symmetrischen aromatischen Harnstoffverbindung der allgemeinen Formel III: 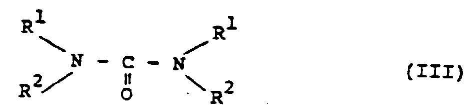 in der R¹ und R² wie vorstehend definiert sind, mit einem Amin der nachstehenden allgemeinen Formel IV: 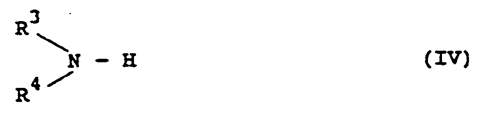 in der R³ und R⁴ wie vorstehend definiert sind,
sowie mit Kohlenmonoxid und Sauerstoff in Gegenwart eines Katalysators umfaßt, der
a) Palladium,
b) einen Organo-Stickstoff- oder Organo-Phosphor-Liganden mit einem einsamen Elektronenpaar und
c) ein Metallsalz enthält, das ein Kation eines Metalls, ausgewählt aus der Gruppe, bestehend aus Kupfer, Eisen, Vanadium, Chrom, Zink, Zinn, Uran und Cer, und ein Anion einer Säure mit einem pK-Wert von weniger als 2, ausgenommen Chlorwasserstoffsäure, umfaßt.

2. Ein Verfahren gemäß Anspruch 1, in dem der Katalysator einen Organo-Stickstoff-Liganden der allgemeinen Formel V: 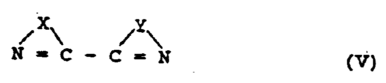 umfaßt, in der X und Y gleiche oder verschiedene Brückengruppen mit jeweils 3 oder 4 Atomen in der Brücke darstellen, wobei von diesen Atomen mindestens zwei Kohlenstoffatome sind, und welche Gruppen X und Y durch eine Verbindung zusätzlich zu derjenigen miteinander verknüpft sein können, die bereits durch die in Formel V dargestellten Kohlenstoffatome gebildet wird.

3. Ein Verfahren gemäß Anspruch 1 oder 2, in dem der Katalysator ein Salz oder ein Komplex von Palladium umfaßt.

4. Ein Verfahren nach irgendeinem der Ansprüche 1 bis 3, in dem das Metallsalz ein Kupferkation umfaßt.

5. Ein Verfahren nach irgendeinem der vorhergehenden Ansprüche, in dem das Metallsalz ein Anion aus der Gruppe umfaßt, die aus Sulfat, Kohlenwasserstoffsulfonaten, Fluorborat und Perchlorat besteht.

6. Ein Verfahren nach Anspruch 5, in dem das Anion das Tosylat ist.

7. Ein Verfahren nach irgendeinem der vorhergehenden Ansprüche, in dem die Reaktion in Gegenwart von Chlorbenzol als Lösungsmittel durchgeführt wird.

8. Ein Verfahren nach irgendeinem der vorhergehenden Ansprüche, in dem der Druck im Bereich von 3 bis 150 Bar liegt.

9. Ein Verfahren nach irgendeinem der vorhergehenden Anspüche, in dem die Temperatur im Bereich von 70 °C bis 160 °C liegt.

10. Ein Verfahren nach irgendeinem der vorhergehenden Ansprüche, in dem das Kohlenmonoxid während der Dauer der Reaktion kontinuierlich in die Reaktionsmischung eingeleitet wird.

11. Ein Verfahren nach irgendeinem der vorhergehenden Ansprüche, in dem das Amin während der Dauer der Reaktion kontinuierlich oder anteilsweise zugespeist wird.

12. Ein Verfahren nach Anspruch 11, in dem das Amin mindestens während 50 % der Dauer der Reaktion eingespeist wird.

## Revendications

1. Procédé de préparation d'urées aromatiques ayant la formule générale I : 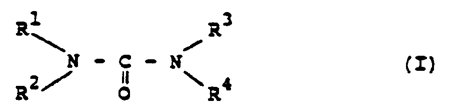 dans laquelle chaque R¹ et R³ représente indépendamment un atome d'hydrogène, ou un groupe alkyle ou cycloalkyle, R² représente un aryle éventuellement substitué ou un groupe hétéroaryle, et R⁴ représente un groupe alkyle, alcényle, alcynyle ou alcoxy, ou un groupe cycloalkyle, aryle ou hétéroaryle, ce procédé comprend de faire réagir une amine aromatique ayant la formule générale II ; 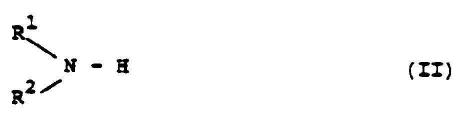 dans laquelle R¹ et R² sont tels que définis ci-dessus, ou une urée aromatique symétrique ayant la formule générale III 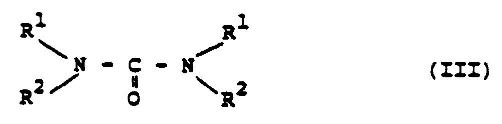 dans laquelle R¹ et R² sont tels que définis ci-dessus, avec une amine ayant la formule générale IV : 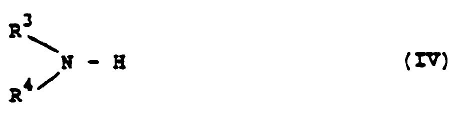 dans laquelle R³ et R⁴ sont tels que définis ci-dessus, avec du monoxyde de carbone en présence d'un catalyseur comprenant :
a) du palladium ;
b) un ligand organo-azote ou organo-phosphore ayant une paire d'électrons isolée ; et
c) un sel métallique comprenant un cation d'un métal choisi dans le groupe constitué du cuivre, fer, vanadium, chrome, zinc, étain, uranium et cérium et un anion d'un acide ayant un pK inférieur à 2, sauf celui d'un acide hydrohalogéné.

2. Procédé selon la revendication 1,dans lequel le catalyseur comprend un ligand organo-azote ayant la formule V 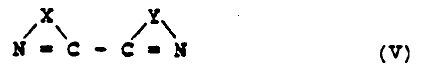 dans laquelle X et Y sont des groupes de pontage identiques ou différents, chacun d'eux ayant 3 ou 4 atomes dans le pont, parmi ceux-ci deux au moins sont des atomes de carbone et les groupes X et Y peuvent être reliés au moyen d'une liaison en plus de celle déjà formée par les atomes de carbone représentés dans la formule V.

3. Procédé selon la revendication 1 ou 2, dans lequel le catalyseur comprend un sel ou complexe de palladium.

4. Procédé selon l'une quelconque des revendications 1 à 3, dans lequel le sel métallique comprend un cation de cuivre.

5. procédé selon l'une quelconque des revendications précédentes, dans lequel le sel de métal comprend un anion du groupe constitué des sulfates, hydrocarbylsulfonates, fluoroborate et perchlorate.

6. Procédé selon la revendication 5, dans lequel l'anion est le tosylate.

7. Procédé selon l'une quelconque des revendications précédentes, dans lequel la réaction est effectuée en présence d'un chlorobenzène comme solvant.

8. Procédé selon l'une quelconque des revendications précédentes, dans lequel la pression est dans l'intervalle de 3 à 150 bar.

9. Procédé selon l'une quelconque des revendications précédentes, dans lequel la température est dans l'intervalle de 70°C à 160°C.

10. Procédé selon l'une quelconque des revendications précédentes, dans lequel on fait passer en continu le monoxyde de carbone dans le mélange réactionnel pendant la durée de la réaction.

11. Procédé selon l'une quelconque des revendications précédentes dans lequel on fait arriver l'amine en continu ou par portions pendant la durée de la réaction.

12. Procédé selon la revendication 11, dans lequel on fait arriver l'amine pendant au moins 50% de la durée de la réaction.
